(19) Europäisches Patentamt
European Patent Office
Office européen des brevets

(11) Publication number: 0 244 399 B1

(12) **EUROPEAN PATENT SPECIFICATION**

(45) Date of publication of patent specification: 30.05.90

(51) Int. Cl.$^5$: C 09 B 17/02 // B41M5/26

(21) Application number: 85905257.3

(22) Date of filing: 07.10.85

(86) International application number: PCT/US85/01920

(87) International publication number: WO 87/02048 09.04.87 Gazette 87/08

# (54) NOVEL PHENAZINE DYES.

(43) Date of publication of application: 11.11.87 Bulletin 87/46

(45) Publication of the grant of the patent: 30.05.90 Bulletin 90/22

(84) Designated Contracting States: DE FR GB IT

(56) References cited:
EP-A-0 124 296
DE-A-2 154 659
GB-A-1 443 403

(73) Proprietor: MINNESOTA MINING AND MANUFACTURING COMPANY
3M Center, P.O. Box 33427
St. Paul Minnesota 55133-3427 (US)

(72) Inventor: MILLER, Alan, G.
P.O. Box 33427
Saint Paul, MN 55133 (US)
Inventor: BALCHUNIS, Robert, J.
P.O. Box 33427
Saint Paul, MN 55133 (US)

(74) Representative: Baillie, Iain Cameron et al
c/o Ladas & Parry Isartorplatz 5
D-8000 München 2 (DE)

EP 0 244 399 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to phenazine dyes, and more particularly, to phenazine dyes suitable as intermediates for leuco phenazine dyes.

Phenazine dyes have been used for many industrial purposes for over 100 years. Well known examples of phenazine dyes include Heliotrope B, and phenosafranine. Essentially all of the phenazine dyes capable of providing red, organge, and magenta hues have substituted amine groups at the 3- or 7-position, or both.

The presence of unsubstituted amine groups at the 3- or 7-positions, or both, does not allow one to employ a simple, direct method to reduce and acylate the dye at the 10-position without also acylating the dye at the 3- or 7-position or both. In a reduced leuco dye which contains acyl groups at the 10-position as well as the 3- and/or 7-positions, reoxidation under normal conditions removes only the acyl group at the 10-position, which will yield a dye form having different absorption properties from that of the original, unreduced dye containing the unsubstituted amine group. Therefore, phenazine dyes known in the art, such as phenosafranine, which are red, magenta, and the like, do not provide the original dye color upon reduction, acylation and reoxidation. In addition, a phenazine dye acylated and reoxidation. In addition, a phenazine dye acylated at the 10-position and containing a free amine group at the 3 and/or 7-positions provides a very unstable leuco dye form in an imaging system containing a metal nitrate, due to the presence of the unsubstituted amine group. Thus, there is no known method for providing phenazine dyes which can be converted to stable leuco forms and then reoxidized to give orange, red and magenta colors in a metal nitrate system. Although phenazine dyes containing substituents at the 3- and 7-positions are well known, essentially all of them provide turquoise or blue colors.

British Patent Specifications 1,443,403 and 1,440,948 describe phenazine dyes for use on cellulosic and other fibers having substituents on the 3- and 7-positions. These substituents are sulfonated phenyl and sulfonated benzyl groups. Although these sulfonated groups provide enhanced water solubility of the dye, they cannot be converted to stable leuco forms that can be oxidized to form orange, red, or magenta colors. British Patent Specification 1,193,923 describes phenazine dyes having long chain alkyl groups attached to the aromatic rings thereof. Although long alkyl chains reduce the mobility of and diffusibility of the dyes, they impart a high molecular weight and low solubility, and they cannot be converted to stable leuco forms that can be oxidized to form orange, red, or magenta colors.

This invention involves 3,7-diamino phenazine dyes having at least one electon-withdrawing substituent on at least one of the groups attached to the nitrogen atoms of the amine groups at the 3- or 7-positions.

These dyes can be used to prepare leuco dyes (stable leuco form) that are capable of being oxidized to provide red, yellow, magenta, and orange colors. The dyes in their leuco form possess sufficient stability to avoid premature oxidation in imaging systems. The oxidized form of the phenazine dyes have good stability to heat and light.

The phenazine dyes of this invention can be represented by the formula:

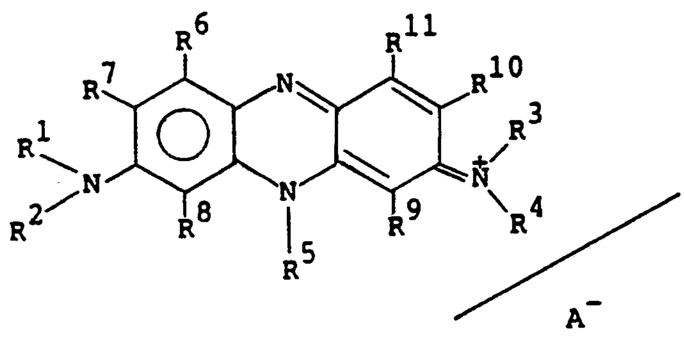

wherein

$R^1$, $R^2$, $R^3$, and $R^4$ independently represent members of the group selected from unsubstituted alkyl groups, substituted alkyl groups, unsubstituted acyl groups, substituted acyl groups, unsubstituted aryl groups, substituted aryl groups, and hydrogen or $R^1$ and $R^2$ or $R^3$ and $R^4$ form a closed ring, provided that at least one of $R^1$, $R^2$, $R^3$, $R^4$ is selected from the group consisting of unsubstituted and substituted acyl groups substituted alkyl groups and substituted aryl groups, provided that at least one of the substituents on the substituted alkyl group or substituted aryl group is an electron-withdrawing substituent;

$R^5$ represents a member of the group selected from unsubstituted alkyl groups, substituted alkyl groups, unsubstituted aryl groups, and substituted aryl groups:

$R^6$, $R^7$, $R^8$, $R^9$, $R^{10}$, and $R^{11}$ independently represent members of the group selected from hydrogen, halogens, unsubstited alkyl groups, substituted alkyl groups, unsubstituted alkoxy groups, and substituted alkoxy groups; and $A^-$ represents any stable anion. e.g., $Cl^-$, or $I^-$.

As used herein, the term "electron-withdrawing substituent" means a group having a Hammett Sigma Parameter (δ) value greater than zero (Lange's Handbook of Chemistry, 12th ed., McGraw-Hill, NY 1979 (pp. 3—134 to 3—137).

Where $R^1$, $R^2$, $R^3$ or $R^4$ is an alkyl group, it is prefered that it contain 1 to 6 carbon atoms. Where $R^1$, $R^2$, $R^3$, or $R^4$ is an aryl group, it is preferred that it be a phenyl or naphthyl group. Where $R^1$, $R^2$, $R^3$, or $R^4$ is an acyl group, i.e.,

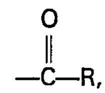

it is preferred that R be an alkyl group having 1 to 10 carbon atoms, a substituted or unsubstituted phenyl group, or a substituted or unsubstituted naphthyl group. When R is a substituted naphthyl or substituted phenyl group, it is preferred that the substituents on the naphthyl group or phenyl group be electron-withdrawing substituents. Where $R^1$, $R^2$, $R^3$, or $R^4$ is a substituted alkyl or aryl group, the substituents are preferably electron-withdrawing. Representative examples of such suitable electron-withdrawing substituents include —Cl, —F, —Br, —CN, —$NO_2$, —OH,

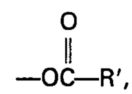

where R′ is an unsubstituted alkyl group having 1 to 10 carbon atoms, a substituted alkyl group having 1 to 10 carbon atoms and preferably being substituted with one or more halogen atoms, unsubstituted aryl groups, e.g., phenyl, naphthyl, or substituted aryl groups, e.g. substituted phenyl, substituted naphthyl, wherein the substituents are preferably halogen atoms or halo-substituted alkyl groups, preferably lower alkyl (1—4 carbon atoms). Where $R^1$, $R^2$, $R^3$, or $R^4$ is an acyl group, the acyl group may contain, but is not required to contain an electron-withdrawing substituent, since the acyl group itself is strongly electro-withdrawing. $R^1$ and $R^2$ groups or the $R^3$ and $R^4$ groups can be joined to form a five or six-membered ring structure, i.e.,

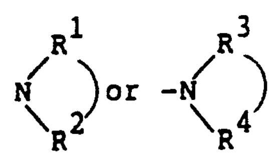

wherein the members forming the ring include nitrogen and carbon atoms, and, optionally oxygen and sulfur atoms. $R^5$ can be an unsubstituted or substituted alkyl group, having, for example 1 to 6 carbon atoms, an unsubstituted or substituted aryl group, having, for example, 4 to 16 carbon atoms in a monocyclic or polylcyclic configuration. Where $R^5$ is an aryl group, it is preferably a phenyl group or a naphthyl group. Representative examples of $R^5$ include the phenyl group, the ethyl group, and the p-methoxy phenyl group. Where $R^6$, $R^7$, $R^8$, $R^9$, $R^{10}$ or $R^{11}$ is an alkyl or an alkoxy group, it is preferred that it contain 1 to 6 carbon atoms. Where $R^6$, $R^7$, $R^8$, $R^9$, $R^{10}$, or $R^{11}$ are substituted alkyl or substituted alkoxy groups, it is preferred that the substituants be halogens. $A^-$ represents any stable anion, preferably $Cl^-$ or $I^-$.

Particularly preferred groups are:

$R^1$ as $NCCH_2CH_2$- and $CF_3CH_2$-; $R^2$ as $NCCH_2CH_2$- and $-CH_3$;

$R^3$ as $NCCH_2$-, $CF_3CH_2$-, $-CH_3$, 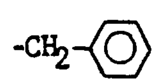 , and H;

$R^4$ as $NCH_2CH_2$-, $-CH_3$, H,

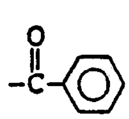 , 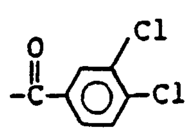 , 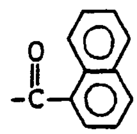 , 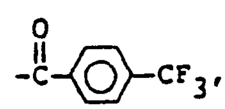

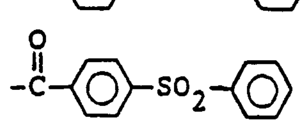 and 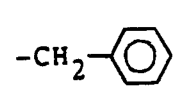 .

Representative examples of phenazine dyes of the present invention are set forth in Table I.

**TABLE I**

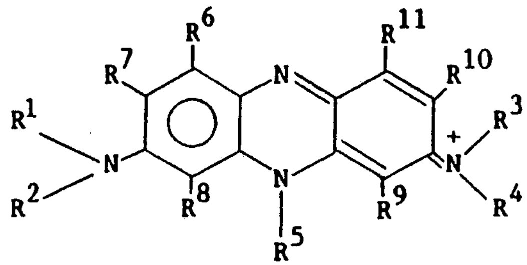

$A^-$

| Dye | $R^1$ | $R^2$ | $R^3$ | $R^4$ | $R^5$ | $R^6,R^7,R^8$ | $R^9$ | $R^{10}$ | $R^{11}$ | A | λ max (nm) |
|---|---|---|---|---|---|---|---|---|---|---|---|
| A | $NCCH_2CH_2-$ | $NCCH_2CH_2-$ | $NCCH_2CH_2-$ | $NCCH_2CH_2-$ | 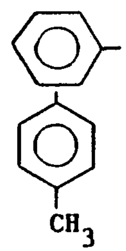 | H | H | H | H | Cl | 561* |
| B | $CF_3CH_2-$ | $-CH_3$ | $CF_3CH_2-$ | $-CH_3$ | | H | H | H | H | I | 547* |
| C | $NCCH_2CH_2-$ | $NCCH_2CH_2-$ | H | H | 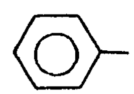 | H | H | H | H | Cl | 543* |
| D | $CF_3CH_2-$ | $NCCH_2CH_2-$ | H | 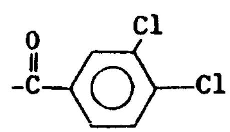 | 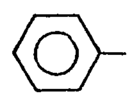 | H | H | H | H | | 514* |

| Dye | $R^1$ | $R^2$ | $R^3$ | $R^4$ | $R^5$ | $R^6,R^7,R^8$ | $R^9$ | $R^{10}$ | $R^{11}$ | A | $\lambda$ max (nm) |
|---|---|---|---|---|---|---|---|---|---|---|---|
| E | $NCCH_2CH_2-$ | $NCCH_2CH_2-$ | H | 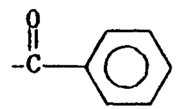 | 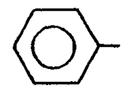 | H | H | H | H | I | 527,561* |
| F | $NCCH_2CH_2-$ | $NCCH_2CH_2-$ | H | 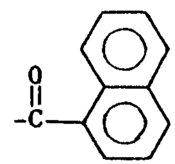 | 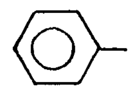 | H | H | H | H | I | 528,544* |
| G | $NCCH_2CH_2-$ | $NCCH_2CH_2-$ | H | 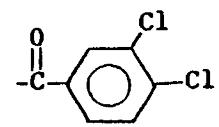 | 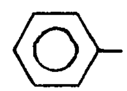 | H | H | H | H | I | 532* |
| H | $NCCH_2CH_2-$ | $NCCH_2CH_2-$ | H | 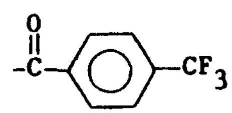 | 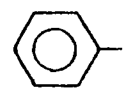 | H | H | H | H | I | 529,544* |
| I | $NCCH_2CH_2-$ | $NCCH_2CH_2-$ | H | 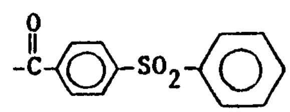 | 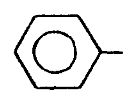 | H | H | H | H | I | 529,544* |
| J | $NCCH_2CH_2-$ | $NCCH_2CH_2-$ | H | H | 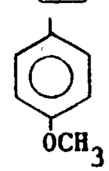 | H | $-CH_3$ | $-CH_3$ | H | I | 532** |

| Dye | $R^1$ | $R^2$ | $R^3$ | $R^4$ | $R^5$ | $R^6,R^7,R^8$ | $R^9$ | $R^{10}$ | $R^{11}$ | A | $\lambda_{max}$ (nm) |
|---|---|---|---|---|---|---|---|---|---|---|---|
| K | $NCCH_2CH_2-$ | $NCCH_2CH_2-$ | $-CH_3$ | $-CH_3$ | 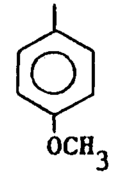 | H | H | H | H | I | 560** |
| L | $NCCH_2CH_2-$ | $NCCH_2CH_2-$ | *** | *** | 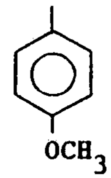 | H | H | H | H | I | 554** |
| M | $NCCH_2CH_2-$ | $NCCH_2CH_2-$ | H | H | 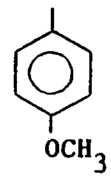 | H | H | $-CH_3$ | H | I | 534** |
| N | $NCCH_2CH_2-$ | $NCCH_2CH_2-$ | H | H | 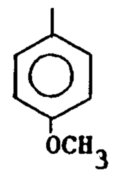 | H | H | H | -Cl | I | 542** |
| O | $NCCH_2CH_2-$ | $NCCH_2CH_2-$ | 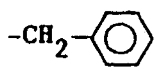 | 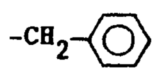 | 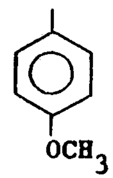 | H | H | H | H | I | 554** |

* measured in saran polymeric binder

** measured in methanol

*** $R^3$ and $R^4$ with N form the ring 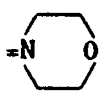

The dyes of the present invention can be colorless when they are in the leuco form, and they can exhibit a red hue, a magenta hue, an orange hue, or a purple hue in their oxidized form. While in the colored form they exhibit excellent resistance to fading or degradation.

The dyes of this invention can be prepared by the process described below. A phenylene diamine derivative, e.g. N,N-diethyl-p-phenylene diamine, an aniline derivative having no para substituent, e.g., N,N-diethylaniline, and an acid, e.g., HCl, are combined in a solvent, preferably water, in a reaction vessel and stirred, preferably at or below room temperature (25°C). An oxidizing agent, preferably sodium dichromate, is added to the solution to couple the phenylene diamine derivative and the aniline derivative. Any aniline derivative containing an unsubstituted $—NH_2$ group or alkylamine compound containing an unsubstituted $—NH_2$ group or alkylamine compound containing an unsubstituted $—NH_2$ group is then added to the mixture. The resulting mixture is then stirred and optionally heated over a sufficient period of time to bring about formation of the dye product. To the resulting solution is added a salt, preferably an ionizable halide salt, e.g., KI, NaCl, to precipitate the dye product. The solution is then cooled, and the dye collected by filtration and dried in air.

In order to prepare dyes of this invention in high yield and high purity the following procedure can be followed:

(1) reacting substantially equimolar amounts of a p-phenylene diamine derivative having one substituted amino group with an aromatic monoamine in the presence of an oxidant in an aqueous acidic environment, such as an aqueous solution of potassium dichromate and hydrochloric acid, preferably at room temperature (about 25°C), although the temperature can vary from −40°C to +80°C,

(2) reacting the intermediate reaction product resulting from the foregoing step with a substantially equimolar amount of a monoamine having one unsubstituted $—NH_2$ group in the presence of an oxidant in an aqueous acidic environment to produce a phenazine dye,

(3) recovering the phenazine dye in pure form by adding excess salt, e.g., halide, fluoroborate, to the product of step (2).

In step (1) of the process, it is important that the p-phenylene diamine derivative and aromatic monoamine be present in substantially equimolar amounts, and that approximately four equivalents of oxidant be present so that formation of undesired by-products is minimized. For the same reason, in step (2) of the process, it is important that the reaction product of step (1) and the monoamine be present in substantially equimolar amounts, and that approximately four equivalents of oxidant be present. An excess of oxidant of up to about 10% in step (1) should have no deleterious effect, however, a deficiency of oxidation in that step is likely to result in an impure intermediate product, which can ultimately result in an impure final product.

One amino group in the p-phenylene diamine derivative should not be substituted. By selecting certain substituents for one of the amino groups of the p-phenylene diamine derivative used in the first step of the process, the substituted amino group can be given electron withdrawing properties, thus determining the required absorption peak of the final dye product. Substitution of the amino group in the aromatic monoamine used in step (1) can also determine the absorption peak. The p-substituent of the aromatic monoamine used in step (2) is preferably electron donating. However, it has little effect on the absorption peak of the resulting dye and its identity is therefore not seriously restricted.

The oxidant for step (1) is preferably selected from the group consisting of alkali metal dichromates, e.g., sodium dichromate. Four equivalents of an oxidant in acidic solution are added, preferably dropwise, to the solution with stirring. The oxidant is preferably selected from the group consisting of sodium dichromate, potassium dichromate, manganese dioxide, bromine, air, and oxygen. The most preferred oxidant is an alkali metal dichromate. It is preferably added in aqueous acid dropwise with stirring to the aqueous acid solution containing the p-phenylene diamine derivative and the aromatic monoamine. The reaction is carried out in an aqueous acid environment wherein the concentration of acid is in the range of 0.1 M to 5 M, and is preferably 0.5 M to 1.0 M. The acid can be any mineral acid or strong monocarboxylic acid, and is preferably selected from the group consisting of hydrochloric acid, sulfric acid, and acetic acid. Because the reaction is exothermic, the reaction temperature can be reduced to maintain control. Monohydric alcohols, e.g., those having 1 to 6 carbon atoms, such as methanol and ethanol, can be used to replace all or part of the aqueous solvent.

In the second step, the oxidants can be milder than those required for the first step. As in the first step, it is important that the ratio between the reactants be approximately equimolar and that approximately four equivalents of oxidant be employed so that the presence of unused reactants in the final product is minimized, thus resulting in a substantially pure final product.

The preferred method is obtaining pure phenazine dye calls for mixing the product of step (2) with an excess (e.g., 2 to 4 times the equimolar amount) of a salt which will precipitate the dye molecule in salt form, such as a halide salt, preferably alkali metal halide, more preferably alkali metal iodide, e.g., sodium iodide, potassium iodide, or a tetrafluoroborate salt, preferably alkali metal tetrafluoroborate. The salt of the dye which forms is insoluble in aqueous acid and precipitates. The salt can then be further purified by recrystallization from an organic solvent, e.g., ethanol or methylene chloride.

The p-phenylene diamine derivative utilized in step (1) is preferably selected from those represented by the structure

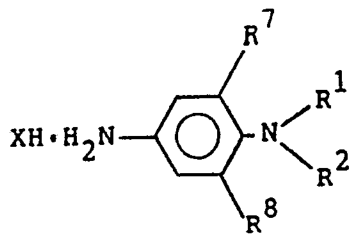

I

wherein

$R^1$ and $R^2$ independently represent a member of the group consisting of alkyl groups, having, for example, 1 to 6 carbon atoms, electron withdrawing groups, for example, cyanoalkyl, haloalkyl, the group where $R^1$ and $R^2$ together complete the closed rings

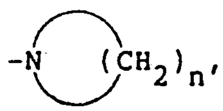

where n is an integer from 4 yo 7, inclusive, and the group where $R^1$ and $R^2$ along with oxygen form the closed ring

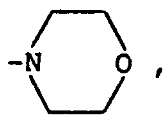

$R^7$ and $R^8$, independently represent a member of the group consisting of hydrogen, halogen, alkyl groups having, for example, 1 to 6 carbon atoms, and

X represents any anion, for example, $Cl^-$,, $SO_4^=$, $Br^-$ and $CH_3COO^-$.

If $R^1$, $R^2$, $R^7$, $R^8$ is an alkyl group, it can contain substituents that do not adversely affect the process of the present invention.

The aromatic monoamine utilized in step (1) is preferably selected from those having the general structures

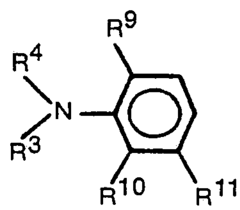

and

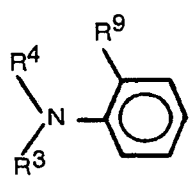

II

wherein

$R^4$ and $R^3$ independently represent a member of the group consisting of hydrogen, alkyl groups having, for example, 1 to 6 carbon atoms, or $R^4$ and $R^3$ together form a closed ring such as

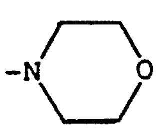 , 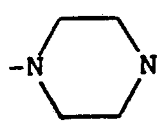 , 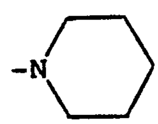 ,

$R^9$, $R^{10}$, and $R^{11}$ represent a member of the group consisting of hydrogen, halogen, alkyl groups having, for example, 1 to 6 carbon atoms, alkoxy groups, having, for example, 1 to 3 carbon atoms, or

If $R^3$, $R^4$, $R^9$, $R^{10}$, $R^{11}$ is an alkyl group it can contain substituents that do not adversely affect the process of the present invention. Examples of preferred substituents for $R^3$ and/or $R^4$ when they are substituted alkyl groups include —Cl, —F, —Br, —CN, —$NO_2$, —OH,

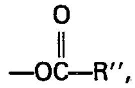

where R'' is an unsubstituted alkyl group having 1 to 10 carbon atoms, a substituted alkyl group having 1 to 10 carbon atoms and preferably being substituted with one or more halogen atoms, unsubstituted aryl groups, e.g., phenyl, naphthyl, or substituted aryl groups, e.g. substituted phenyl, substituted naphthyl, wherein the substituents are preferably halogen atoms or halo-substituted alkyl groups, preferably lower

alkyl (1—4 carbon atoms). If $R^9$, $R^{10}$, $R^{11}$ is an alkoxy group, it can contain substituents that do not adversely affect the process of the present invention.

In the second step of the process, to the solution of dye III can be added a substantially equimolar quantity of an aromatic amine having the structure

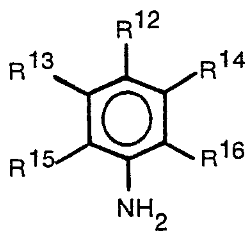

wherein

$R^{12}$ represents a member of the group consisting of hydrogen, halogen, alkyl group having, for example, from 1 to 6 carbon atoms, alkoxy group having, for example, from 1 to 6 carbon atoms, —NR'R'', where R' and R'' independently represent alkyl groups having, for example, from 1 to 6 carbon atoms,

$R^{13}$, $R^{14}$, $R^{15}$, and $R^{16}$ independently represent a member of the group consisting of hydrogen, halogen, and alkyl group having, for example, from 1 to 6 carbon atoms, or

$R^{14}$, $R^{16}$ together form up to 2 condensed rings, or

$R^{12}$, $R^{14}$ together form up to 2 condensed rings, provided that of $R^{13}$, $R^{14}$, $R^{15}$, and $R^{16}$ are hydrogen, it is preferable that $R^{12}$ not be hydrogen.

The aromatic monoamine used in the first step of the process can also be used in the second step. Primary alkyl amines having up to 20 carbon atoms can be used in the second step of the process of this invention.

This process for preparing 3,7-diamine-5-aryl-phenazine dyes according to this invention results in dyes of high purity, e.g., less than about 5% impurity, and in high yield, e.g., as high as 78%, based on p-phenylene diamine derivative.

The dyes of the present invention can be used to prepare leuco dyes that are useful in thermographic and photothermographic imaging systems. They are particularly useful for preparing leuco dyes to be used in the preparation of transparencies for overhead projection because the dyes can exhibit long shelf-life in the unoxidized, colorless state and further exhibit excellent heat and light stability in the oxidized, colored state.

The leuco forms of the dyes of this invention can be prepared in the following manner. The dye of the present invention is dissolved in water, methylene chloride is added, and the pH is adjusted to about 10. In an inert atmosphere, the dye is reduced with sodium dithionate. Acylation can be effected by adding an appropriate acid chloride. When the reaction is complete, the methylene chloride layer is isolated and treated with a decolorizing agent, e.g., Attapulgus clay. Removal of solvent yields the leuco form of the dye.

The following non-limiting examples will further illustrate the invention.

## Example 1

Synthesis of 3-amino-5-phenyl-7-bis(2-cyanoethyl)amino phenazinium chloride (Dye C of Table I)

A 3-liter round-bottomed flask fitted with a mechanical stirrer was loaded with 16.6 g (0.066 mole) of 4-(bis-(2-cyanoethyl)amino) aniline in 800 ml of distilled water. A 10% excess of aniline (13.56 g, 0.1456 mole) and 200 ml of distilled water were added to the mixture. The mixture was cooled to 0°C in an ice bath, and 10 g concentrated hydrochloric acid in 25 ml of water were added. Then 6.31 g (0.0883 mole) of sodium dichromate in 25 ml of water was added. The temperature rose to 7°C. Stirring was continued as 9 ml of concentrated hydrochloric acid in 25 ml of distilled water was added over a period of two hours. After 16 hours the temperature had risen to 20°C. The mixture was heated under reflux for 4 hours and then filtered hot. The filter cake was washed with 1.5 liters of boiling water. The combined filtrates were concentrated to 1.4 liters and then heated to 75°C as 200 g of sodium chloride were added.

The mixture was cooled to room temperature, chilled in an ice bath, and the solid was then recovered by filtration to give 14.651 g (yield = 51.6%). ($\lambda$max=537 nm in methanol).

## Example 2

Synthesis of 3-amino-7-bis(2-cyanoethyl)amino-2,4-di-methyl-5-p-methoxyphenylphenaziniumiodide (Dye J of Table I)

An Erlenmeyer flask was loaded with 1.0 g (0.004 mole) of N,N-bis(2-cyanoethyl)-p-phenylenediamine hydrochloride, 1.0 g concentrated hydrochloric acid, 0.5 g (0.004 mole) 2,6-dimethylaniline, and 100 ml methanol. As the resulting mixture was being stirred at room temperature, 0.8 g (0.0027 mole) of sodium dichromate dihydrate ($Na_2Cr_2O_7$ $2H_2O$) in 3 g $H_2O$ was added dropwise. After two minutes, 0.5 g (0.004 mole of p-anisidine in 3 g methanol was added. After about 20 minutes, an additional 0.5 g (0.0017 mole) of $Na_2Cr_2O_7$ $2H_2O$ in 3 g $H_2O$ was added. After the mixture was stirred for about 1 hour, the solution, which

was dark red in color, was reduced in volume and 50 ml of water was added. The solution was heated, and the liquid decanted. Water (50 ml) was added to the residue, and this solution was heated, and the water decanted. The combined aqueous solutions were treated with diatomaceous earth (Celite®) and filtered hot. To the hot solution was added 5.0 g of potassium iodide to precipitate the dye in the form of salt. The solution was cooled, the dye recovered by filtration, and dried in air. The yield was 1.8 g (78%). The wave lengths were as follows:

λmax = 533 nm in $H_2O$
λmax = 532 nm in $CH_3OH$
λmax = 529 nm in $CH_2Cl_2$

Example 3

Preparation of 3-(4-(phenylsulfonyl)-benzamido)-5-phenyl-7-bis(2-cyanoethyl)amino-5,10-dihydro-10 (4-(phenylsulfonyl)benzoyl)phenazine from Dye C of Table I)

This example demonstrates preparation of a leuco dye suitable for use in a thermally imageable composition.

A 1-liter 3-necked flask was fitted with a Claisen head with two dropping funnels, a mechanical stirrer, and a pH electrode. A solution containing 5.0 g (0.012 mole) of the dye prepared in Example 1, 210 ml of water and 0.2 g of ethylene diamine tetraacetic acid was added to the flask and stirred, while 256 ml of methylene chloride was added. The system was closed amd flushed with argon, the pH adjusted to 10, then 2.44 g (0.014 mole) of sodium dithionite was added. The solution turned orange and the pH dropped to 3.7. Aqueous sodium hydroxide solution (25%) was added to bring the pH to 4.5, and then 7.53 g (0.027 mole) of 4-phenyl sulfonyl benzoyl chloride in 60 ml of methylene chloride was added dropwise. The pH was raised to 10—11. After an additional 50 minutes, the methylene chloride layer was removed and dried over calcium sulfate. The solution was treated twice with 10 g of Attapulgus clay and filtered. The solvent was then evaporated, leaving 8.14 g of solid (79% yield).

The thus-prepared leuco dye was tested in the following formulation:

0.19 g leuco dye
0.75 g (1%) phenidone in tetrahydrofuran
0.13 g (5%) catechol in ethanol
0.075 g phthalic acid
3.0 g tetrahydrofuran
0.13 g $Ni(NO_3)_2$
0.25 g phenol
8.0 g 15% polyvinylidene chloride (Saran® 220) in methyl ethyl ketone

The solution was coated at 3 mils (75 μm) on 4 mil (100 μm) polyester film and dried at 160°F for 2 minutes. The dried film was imaged at a rate of 1.7 inches per second on a 3M Model 45 infrared transparency film machine. Image stability was tested on the stage of a 3M Model 66 projector. The temperature on the stage was 63°C. The results are shown in Table II.

TABLE II

Time on stage of Model 66 Projector

Optical Density

| | 0 hour | | 3 hours | | 6 hours | | 14 hours | |
|---|---|---|---|---|---|---|---|---|
| Filter | image | back-ground | image | back-ground | image | back-ground | image | back-ground |
| yellow | .49 | .07 | .46 | .08 | .47 | .09 | .47 | .10 |
| red | .11 | .04 | .10 | .04 | .10 | .05 | .10 | .05 |
| green | .85 | .07 | .81 | .08 | .82 | .09 | .82 | .11 |
| blue | .53 | .09 | .53 | .09 | .53 | .09 | .54 | .10 |

The high optical density readings of the image with the green and blue filters show that red is the predominant hue of the dye. The λmax is 540 nm as compared with λmax of 587 nm of Heliotrope B. The retention of these density readings after 14 hours on the stage of the projector attest to the light and heat stability of the dye; the retention of the low optical density readings of the background attest to stability of the leuco compound which is the dye precursor.

Example 4

Synthesis of 3,7-di(N-methyl,N-2,2,2-trifluoroethyl)-amino-5-(4-methylphenyl) phenazinium chloride (Dye B of Table I)

Into a 1-liter 3-necked flask equipped with a stirrer was added p-(N-methyl-N-2,2,2-trifluoroethyl)-aminoaniline (1.40 g, 0.0058 M) and N-methyl-N-2,2,2-trifluoroethylaniline (0.764 g, 0.0041 M) with 250 ml of deionized water. The mixture was stirred vigorously as 1.55 g of concentrated hydrochloric acid in 10 ml of water was added. After 10 minutes, 2.31 g of sodium dichromate (0.0078 M) in 20 ml of water was added over a period of several minutes. After 5 minutes, p-toluidine was added (0.624 g, 0.0058 M); over the next 45 minutes, 1.2 g of concentrated hydrochloric acid was added. The solution was warmed to 42°C and stirred overnight at this temperature. After being heated to reflux temperature for 22.5 hours, the mixture was filtered hot, and the filter cake washed with hot water. The aqueous filtrate was concentrated from 650 ml to 275 ml in a rotary vacuum dryer.

The procedure for the reduction was that described in Example 2, using p-phenylsulfonylbenzoyl chloride as the acylating agent. The λmax of the oxidized dye was 547.7 nm; its color was perceptibly more red than that of Heliotrope B (λmax −587 nm).

The leuco dye was evaluated in a formulation identical to that of Example 3 except for the use of a different dye. The procedure for evaluation was the same as that employed in Example 3. The film exhibited imaging characteristics as shown in the following table.

TABLE III

| | Optical Density | |
|---|---|---|
| Filter | image | background |
| yellow | .38 | .04 |
| red | .08 | .03 |
| green | 1.10 | .05 |
| blue | .48 | .07 |

From the foregoing table, it can be seen that the dye in the oxidized form is red in color.

Various modifications and alterations of this invention will become apparent to those skilled in the art without departing from the the scope and spirit of this invention, and it should be understood that this invention is not to be unduly limited to the illustrative embodiments set forth herein.

**Claims**

1. Phenazine dye represented by the formula:

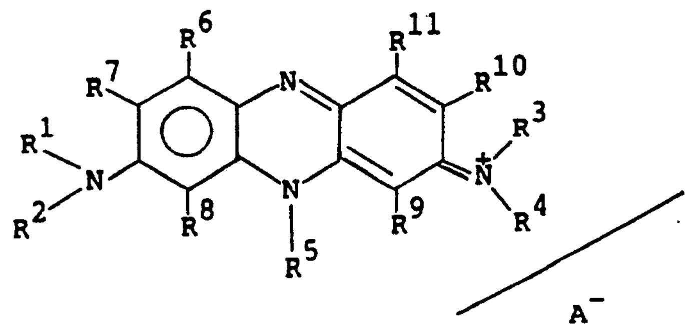

wherein

$R^1$, $R^2$, $R^3$, and $R^4$ independently represent a member selected from the group consisting of unsubstituted alkyl groups, substituted alkyl groups, unsubstituted acyl groups, substituted acyl groups, unsubstituted aryl groups, substituted aryl groups, and hydrogen or $R^1$ and $R^2$ or $R^3$ and $R^4$ form a closed ring, provided that at least one of $R^1$, $R^2$, $R^3$, and $R^4$ is selected from the group consisting of unsubstituted and substituted acyl groups, substituted alkyl groups, and substituted aryl groups, provided that at least one of the substituents on the substituted alkyl group or substituted aryl group is an electron-withdrawing substituent having a Hammet Parameter (σ) value greater than zero,

$R^5$ represents a member selected from the group consisting of unsubstituted alkyl groups, substituted alkyl groups, unsubstituted aryl groups, and substituted aryl groups;

$R^6$, $R^7$, $R^8$, $R^9$, $R^{10}$, and $R^{11}$ independently represent members of the group selected from hydrogen, halogens, unsubstituted alkyl groups, substituted alkyl groups, unsubstituted alkoxy groups, and substituted alkoxy groups; and

$A^-$ represents any stable anion, said dye being capable of being converted to stable leuco form and being capable of being subsequently re-oxidized to give an orange, red, yellow or magenta colour.

2. A dye according to claim 1 wherein $R^1$ is selected from the group consisting of $NCCH_2CH_2$— and $CF_3CH_2$—.

3. A dye according to claim 1 or claim 2 wherein $R^2$ is selected from the group consisting of $NCCH_2CH_2$— and —$CH_3$.

4. A dye according to any preceding claim wherein $R^3$ is selected from the group consisting of

$NCCH_2CH_2-$, $CF_3CH_2-$, $-CH_3$, 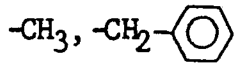 , and H.

5. A dye according to any preceding claim wherein $R^4$ is selected from the group consisting of

$NCH_2CH_2-$, $-CH_3$, H,

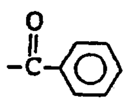 , 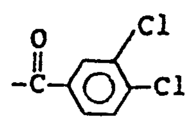 , 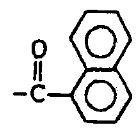 , 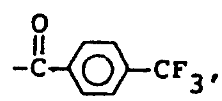

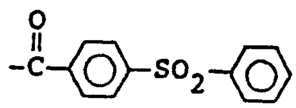 and 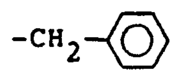 .

6. A dye according to any preceding claim wherein $R^5$ is selected from the group consisting of phenyl, methoxyphenyl and methylphenyl.

7. A dye according to any preceding claim wherein A is $Cl^-$ or $I^-$.

8. A process for preparing phenazine dyes according to claim 1 comprising the steps of

(1) reacting, in an aqueous acidic environment on the presence of approximately 4 equivalents of an oxidant, substantially equimolar amounts of a p-phenylene diamine derivative and an aromatic monoamine having one unsubstituted —$NH_2$ group,

(2) reacting, in an aqueous acidic environent in the presence of approximately 4 equivalents an oxidant, substantially equimolar amounts of the product of step (1) and a monoamine, and

(3) recovering a substantially pure phenazine dye by adding a salt to precipitate the dye.

9. A process according to claim 8 wherein the p-phenylene diamine derivative is represented by the following structure:

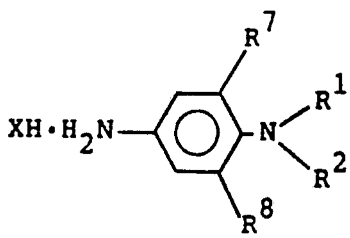

wherein

$R^1$ and $R^2$ independently represent alkyl groups or electron withdrawing groups, or

$R^1$ and $R^2$ together represent the closed rings

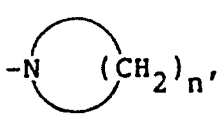

where n represents an integer from 4 to 7 inclusive, or

$R^1$ and $R^2$ together represent the closed ring

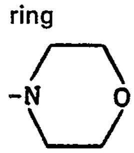 ,

$R^7$ and $R^8$ independently represent hydrogen, halogen, optionally substituted alkyl or optionally substituted alkoxy,

X represents an anion.

10. A process according to claim 8 or claim 9 wherein the aromatic monoamine in step (1) is represented by the following structure:

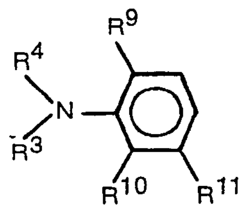

or

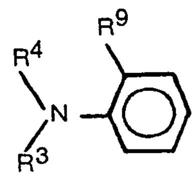

II

wherein $R^3$, $R^4$, $R^9$, $R^{10}$ and $R^{11}$ are as defined in claim 1.

11. A process according to any one of claims 8 to 10 wherein the monoamine of step (2) is represented by the following structure:

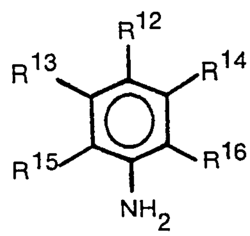

wherein

$R^{12}$ represents a member of the group consisting of hydrogen, halogen, alkyl, alkoxy, —NR′ R″ where R′ and R″ are alkyl groups,

$R^{13}$, $R^{14}$, $R^{15}$ and $R^{16}$ independently represent a member of the group consisting of hydrogen, halogen and alkyl, or $R^{14}$, and $R^{16}$ together form up to 2 condensed rings or $R^{12}$ and $R^{14}$ together form up to 2 condensed rings.

12. A process according to any of claims 8 to 10 wherein the monoamine of step (2) is a primary alkyl amine having up to 20 carbon atoms.

13. A process according to any of claims 8 to 10 wherein the monoamine of step (2) is a represented by the following structure:

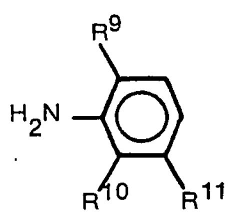

or

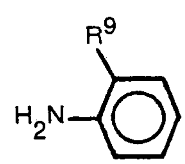

wherein

$R^9$, $R^{10}$ and $R^{11}$ are as defined in claim 1.

14. A process according to any of claims 8 to 13 wherein the recovery step is conducted by adding halide salt or tetrafluoroborate salt to precipitate the dye.

**Patentansprüche**

1. Phenazinfarbstoff, der durch die Formel

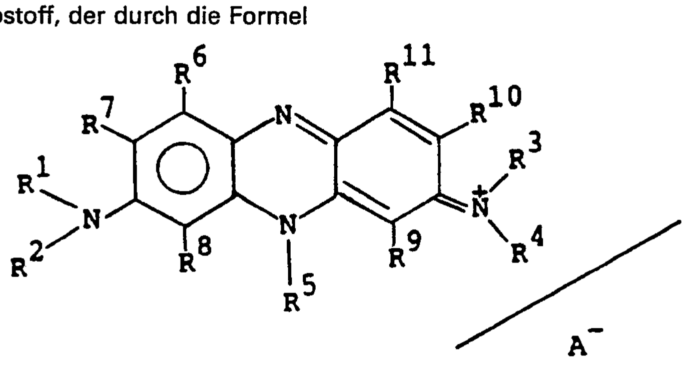

dargestellt ist, in der $R^1$, $R^2$, $R^3$ und $R^4$ unabhängig voneinander ein Glied darstellen, das aus der Gruppe ausgewählt ist, die aus den nichtsubstituierten Alkylgruppen, substituierten Alkylgruppen, nichtsubstituierten Acylgruppen, substituierten Acylgruppen, nichtsubstituierten Arylgruppen, substituierten Arylgruppen und Wasserstoff besteht oder $R^1$ und $R^2$ oder $R^3$ und $R^4$ einen geschlossenen Ring bilden, sofern mindestens eines der Glieder $R^1$, $R^2$, $R^3$, $R^4$ aus der Gruppe ausgewählt ist, die aus den nichtsubstituierten und substituierten Acylgruppen, substituierten Alkylgruppen und substituierten Arylgruppen besteht, sofern mindestens einer der Substituenten der substituierten Alkylgruppe oder der substituierten Arylgruppe ein elektronenanziehender Substituent ist, bei der Hammet-Sigma-Parameter ($\sigma$) größer ist als null;

$R^5$ ein Glied darstellt, das aus den der Gruppe ausgewählt ist, die aus nichtsubstituierten Alkylgruppen, substituierten Alkylgruppen, nichtsubstituierten Arylgruppen und substituierten Arylgruppen besteht;

$R^6$, $R^7$, $R^8$, $R^9$, $R^{10}$ und $R^{11}$ unabhängig voneinander Glieder darstellen, die aus der Gruppe ausgewählt sind, die aus Wasserstoff, den Halogenen, nichtsubstituierten Alkylgruppen, substituierten Alkylgruppen, nichtsubstituierten Alkoxygruppen und substituierten Alkoxygruppen besteht; und

$A^-$ ein beliebiges stabiles Anion darstellt, wobei der Farbstoff in eine stabile Leukoform konvertierbar ist und danach rückoxidierbar ist, so daß er eine orangefarbene, rote, gelbe oder magentafarbene Färbung ergibt.

2. Farbstoff nach Anspruch 1, dadurch gekennzeichnet, daß $R^1$ aus der Gruppe ausgewählt ist, die aus $NCCH_2CH_2$— und $CF_3CH_2$— besteht.

3. Farbstoff nach Anspruch 1 oder 2, dadurch gekennzeichnet, daß $R^2$ aus der Gruppe ausgewählt ist, die aus $NCCH_2CH_2$— und $CH_3$ besteht.

4. Farbstoff nach einem der vorhergehenden Ansprüche, dadurch gekennzeichnet, daß $R^3$ aus der Gruppe ausgewählt ist, die aus $NCCH_2CH_2$— $CF_3CH_2$—, —$CH_3$,

$NCCH_2CH_2$-, $CF_3CH_2$-, -$CH_3$, -$CH_2$- 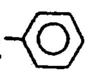 , H und besteht.

5. Farbstoff nach einem der vorhergehenden Ansprüche, dadurch gekennzeichnet, daß $R^4$ aus der Gruppe ausgewählt ist, die aus

$NCH_2CH_2$-, -$CH_3$, H,

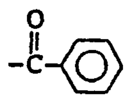 , 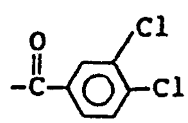 , 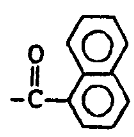 , 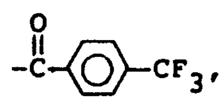 ,

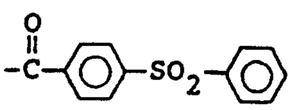 und 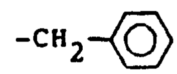 .

besteht.

6. Farbstoff nach einem der vorhergehenden Ansprüche, dadurch gekennzeichnet, daß $R^5$ aus der Gruppe ausgewählt ist, die aus Phenyl, Methoxyphenyl und Methylphenyl besteht.

7. Farbstoff nach einem der vorhergehenden Ansprüche, dadurch gekennzeichnet, daß A $Cl^-$ oder $O^-$ ist.

8. Verfahren zum Erzeugen von Phenazinfarbstoffen nach Anspruch 1 mit folgenden Schritten:

(1) in einem wäßrigen sauren Medium werden in Gegenwart von etwa 4 Äquivalentmassen eines Oxidationsmittels im wesentlichen äquimolare Mengen eines p-Phenylendiaminderivats und eines aromatischen Monoamins, das eine nichtsubstituierte —$NH_2$-Gruppe hat, umgesetzt,

(2) in einem wäßrigen sauren Medium werden in Gegenwart von etwa 4 Äquivalentmassen eines Oxidationsmittels im wesentlichen äquimolare Mengen des Produkts des Schritts (1) und ein Monoamin umgesetzt und

(3) durch Zugabe eines Salzes zum Ausfällen eines Phenazinfarbstoffes wird dieser in im wesentlichen reiner Form gewonnen.

9. Verfahren nach Anspruch 8, dadurch gekennzeichnet, daß das p-Phenylendiaminderivat durch die Struktur

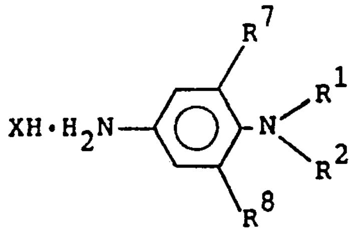

dargestellt ist, in der

$R^1$ und $R^2$ unabhängig voneinander Alkylgruppen oder elektronenanziehende Gruppen darstellen oder

$R^1$ und $R^2$ zusammen die geschlossenen Ringe

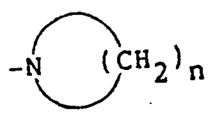

darstellen, in denen n eine ganze Zahl von 4 bis 4 bis einschließlich 7 ist, oder

$R^1$ und $R^2$ zusammen mit Sauerstoff den geschlossenen Ring

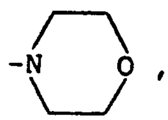 ,

darstellen,

$R^7$ und $R^8$ unabhängig voneinander Wasserstoff, Halogen, ein gegebenefalls substituiertes Alkyl oder ein gegebenenfalls substituiertes Alkoxy darstellen und

X ein Anion darstellt.

10. Verfahren nach Anspruch 8 oder 9, dadurch gekennzeichnet, daß das im Schritt (1) verwendete aromatische Monoamin durch die Struktur

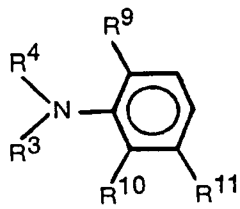 oder 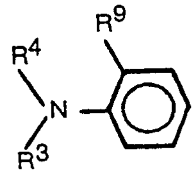 II

dargestellt ist, in der $R^3$, $R^4$, $R^9$, $R^{10}$ und $R^{11}$ die im Anspruch 1 angegebenen Bedeutungen haben.

11. Verfahren nach einem der Ansprüche 8 bis 10, dadurch gekennzeichnet, daß das im Schritt (2) verwendete Monoamin durch die Struktur

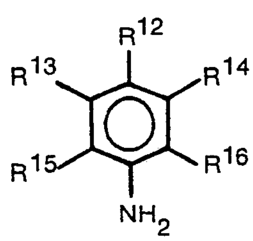

dargestellt ist, in der

$R^{12}$ ein Glied der Gruppe darstellt, die aus Wasserstoff, Halogen, Alkyl, Alkoxy und —NR'R'' besteht, wobei

R' und R'' Alkylgruppen sind, und $R^{13}$, $R^{14}$, $R^{15}$ und $R^{16}$ unabhängig voneinander ein Glied der Gruppe darstellen, die aus Wasserstoff, Halogen und Alkyl besteht, oder $R^{14}$ und $R^{16}$ zusammen bis zu 2 kondensierte Ringe bilgen oder $R^{12}$ und $R^{14}$ Zussammen bis zu 2 kondensierte Ringe bilden.

12. Verfahren nach einem der Ansprüche 8 bis 10, dadurch gekennzeichnet, daß das im Schritt (2) verwendendete Monoamin ein Primäralkylamin mit bis zu 20 Kohlenstoffatomen ist.

13. Verfahren nach einem der Ansprüche 8 bis 10, dadurch gekennzeichnet, daß das im Schritt (2) verwendete Monoamin ein durch die Struktur

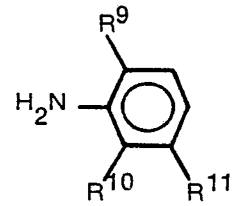

oder

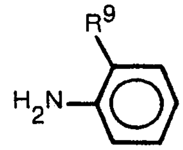

dargestellt ist,

in der $R^9$, $R^{10}$ und $R^{11}$ die im Anspruch 1 angegebenen Bedeutungen haben.

14. Verfahren nach einem der Ansprüche 8 bis 13, dadurch gekennzeichnet, daß in dem Gewinnungsschritt der Farbstoff durch Zugabe eines Halogenidsalzes oder eines Tetrafluoroboratsalzes ausgefällt wird.

**Revendications**

1. Colorant phénazinique représenté par la formule suivante:

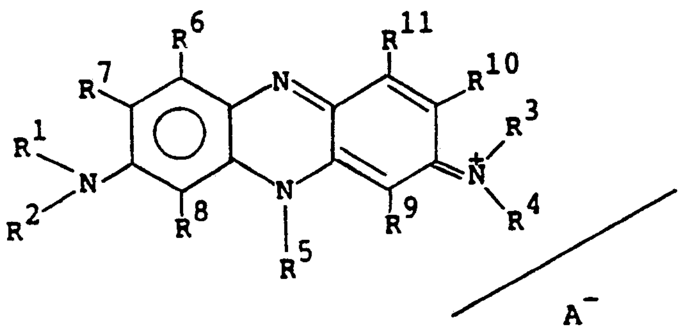

dans laquelle

$R^1$, $R^2$, $R^3$ et $R^4$, indépendamment les uns des autres, représentent chacun un radical choisi parmi les groupes alkyle non-substitués, les groupes alkyle substitués, les groupes acyle non-substitués, les groupes acyle substitués, les groupes aryle non-substitués, les groupes aryle substitués, et l'hydrogène, ou encore $R^1$ et $R^2$, ou $R^3$ et $R^4$, forment un cycle fermé, à la condition qu'au moins l'un des radicaux $R^1$, $R^2$, $R^3$, $R^4$ soit choisi parmi l'ensemble comprenant les groupes acyle non-substitués et substitués, les groupes alkyle substitués et les groupes aryle substitués, à la condition qu'au moins l'un des substituants du groupes alkyle substitué ou du groupe aryle substitué soit un substituant attracteur d'électrons présentant un paramètre Sigma de Hammet ($\sigma$) supérieur à zéro;

$R^5$ représente un radical choisi parmi l'ensemble comprenant les groupes aryle non-substitués, les groupes alkyle substitués, les groupes aryle non-substitués et les groupes aryle substitués;

$R^6$, $R^7$, $R^8$, $R^9$, $R^{10}$ et $R^{11}$, indépendamment les uns des autres, représentent chacun un radical choisi parmi l'ensemble comprenant l'hydrogène, les halogènes, les groupes alkyle non-substitués, les groupes alkyle substitués, les groupes alcoxy non-substitués et les groupes alcoxy substitués; et

$A^-$ représente un anion stable quelconque, ledit colorant pouvant être converti en une forme leucodérivée stable et étant capable de subir ultérieurement une réoxydation pour donner une couleur orangée, rouge jaune ou magenta.

2. Colorant selon la revendication 1, dans lequel $R^1$ est choisi parmi l'ensemble comprenant $NCCH_2CH_2$— et $CF_3CH_2$—.

3. Colorant selon la revendication 1 ou la revendication 2, dans lequel $R^2$ est choisi parmi l'ensemble comprenant $NCCH_2CH_2$— et —$CH_3$.

4. Colorant selon l'une quelconque des revendications précédentes, dans lequel $R^3$ est choisi parmi l'ensemble comprenant

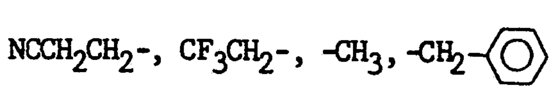, et H.

5. Colorant selon l'une quelconque des revendications précédentes, dans lequel $R^4$ est choisi parmi l'ensemble comprenant $NCCH_2CH_2$—, —$CH_3$, H.

$NCH_2CH_2-$, $-CH_3$, H,

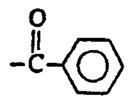 , 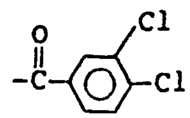 , 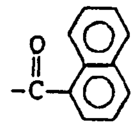 , 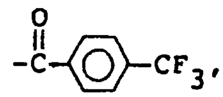

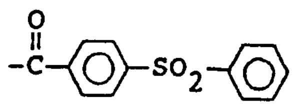 et 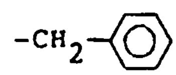 .

6. Colorant selon l'une quelconque des revendications précédentes, dans lequel $R^5$ est choisi parmi l'ensemble comprenant les radicaux phényle, méthoxyphényle et méthylphényle.

7. Colorant selon l'une quelconque des revendications précédentes, dans lequel A est $Cl^-$ ou $I^-$.

8. Procédé de préparation de colorants phénaziniques selon la revendication 1, comprenant les étapes consistant:

(1) à faire réagir, dans un environnement acide aqueux et en présence d'environ 4 équivalents d'un oxydant, des quantités pratiquement équimolaires d'un dérivé de la p-phénylènediamine et une monoamine aromatique comportant un groupe $—NH_2$ non-substitué,

(2) à faire réagir, dans un environnement acide aqueux et en présence d'environ 4 équivalents d'un oxydant, des quantités pratiquement équimolaires du produit de l'étape (1) et d'une monoamine, et

(3) à récupérer un colorant phénazinique pratiquement pur par addition d'un sel pour provoquer la précipitation du colorant.

9. Procédé selon la revendication 8, dans lequel le dérivé de la p-phénylènediamine est représenté par la formule développée suivant:

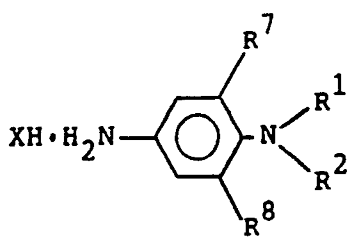

dans laquelle

$R^1$ et $R^2$, indépendamment l'un de l'autre, représentent chacun un groupe alkyle ou un groupe attracteur d'électrons, ou bien

$R^1$ et $R^2$ forment ensemble les cycles fermés

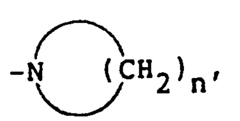

où n représente un nombre entier de 4 à 7, limites comprises, ou encore

$R^1$ et $R^2$, avec l'oxygène, représentent le cycle fermé

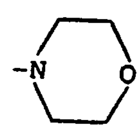 ,

$R^7$ et $R^8$, indépendamment l'un de l'autre, représentent chacun un hydrogène, un halogène, un radical alkyle facultativement substitué ou alcoxy facultativement substitué,

X représente un anion.

10. Procédé selon la revendication 8 ou la revendication 9, dans lequel la monoamine aromatique de l'étape (1) possède la formule développée suivante:

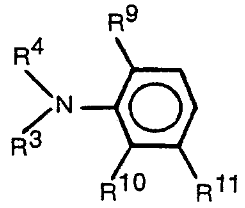

ou

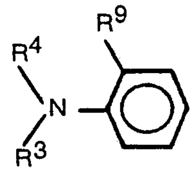

II

où $R^3$, $R^4$, $R^9$, $R^{10}$ et $R^{11}$ sont tels que définis dans la revendication 1.

11. Procédé selon l'une quelconque des revendications 8 à 10, dans lequel la monoamine de l'étape (2) est représentée par la formule développée suivante:

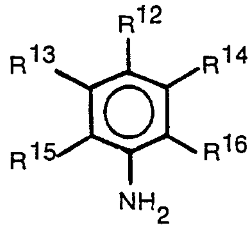

dans laquelle

$R^{12}$ représente un radical de l'ensemble comprenant l'hydrogène, les halogènes, les radicaux alkyle alcoxy, —NR'R'' où R' et R'' sont des groupes alkyle,

$R^{13}$, $R^{14}$, $R^{15}$ et $R^{16}$, indépendamment les uns des autres, représentent chacun un radical choisi dans l'ensemble comprenant l'hydrogène, les halogènes et les radicaux alkyle, ou encore $R^{14}$ et $R^{16}$ forment ensemble jusqu'à 2 noyaux condensés, ou encore $R^{12}$ et $R^{14}$ forment ensemble jusqu'à 2 noyaux condensés.

12. Procédé selon l'une quelconque des revendications 8 à 10, dans lequel la monoamine de l'étape (2) est une alkylamine primaire ayant jusqu'à 20 atomes de carbone.

13. Procédé selon l'une quelconque des revendications 8 à 10, dans lequel la monoamine de l'étape (2) est représentée par la formule développée suivante:

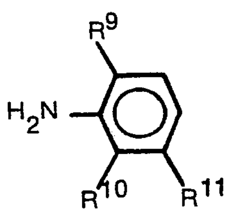

ou

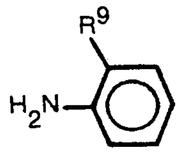

où $R^9$, $R^{10}$ et $R^{11}$ sont tels que définis dans la revendication 1.

14. Procédé selon l'une quelconque des revendication 8 à 13, dans lequel l'étape récupération est mise en oeuvre par addition d'une halogenure ou d'un tétrafluoroborate pour provoquer la précipitation du colorant.